# EUROPEAN PATENT SPECIFICATION

(11) **EP 3 222 336 B1**
(45) Date of publication and mention of the grant of the patent: **27.02.2019**
(21) Application number: 16161388.0
(22) Date of filing: 21.03.2016
(51) Int. Cl.: C12M 1/00, C12M 1/107, B01D 21/00, B01D 21/24

(54) **TANK FOR SEDIMENTATION OF THE INORGANIC PART OF POULTRY DEJECTION AND RELATIVE METHOD FOR REMOVING SAID INORGANIC PART**
TANK ZUR SEDIMENTATION DES ANORGANISCHEN TEILS VON GEFLÜGELAUSSCHEIDUNGEN UND ZUGEHÖRIGES VERFAHREN ZUM ENTFERNEN DES ANORGANISCHEN TEILS
RÉSERVOIR POUR LA SÉDIMENTATION DE LA PARTIE INORGANIQUE DE DÉJECTIONS DE VOLAILLES ET PROCÉDÉ ASSOCIÉ POUR L'ÉLIMINATION DE LADITE PARTIE INORGANIQUE

(43) Date of publication of application: 27.09.2017
(73) Proprietor: Green Seed S.r.l., 24121 Bergamo (BG) (IT)
(72) Inventor: CAIMI, Sergio Renato, I-24121 Bergamo BG (IT)
(74) Representative: Ercolani, Simone Pietro

(56) References cited:
- GB-A- 914 393
- US-A- 5 782 950
- US-A1- 2013 087 515

## Description

### Field of the Invention

The present invention concerns a tank for settling the inorganic portion of the avicultural dejections and respective method of removing said inorganic portion. In particular, such a tank is used in plants for producing biogas or biomethan from digested organic material of the type just coming from the avicultural dejections.

### Background Art

Such plants for producing biogas or biomethan are always provided with upstream tanks preliminarily allowing the separation of the organic portion useful for the following plant operation, from that one containing calcium carbonate in which such avicultural dejections are rich. In fact, it is known that farm feedstuffs of laying hens are rich in calcium carbonate useful for aiding the egg production. Such calcium carbonate is then highly concentrated in avicultural dejections and can cause malfunctions and efficiency losses of plants for the anaerobic digestion of such organic materials.

According to the prior art, common tanks arranged upstream of the plant for producing biogas or biomethan are used just for separating the organic portion of the avicultural dejections from the inorganic one, by settling by gravity the latter on the tank bottom. Such tanks comprise a settling chamber to settle the organic material, usually in granular solid form, contained in said avicultural dejections and suctioning means to suction such a settled granular solid material. In particular, the suctioning means comprise a suction duct arranged on the chamber bottom and, if need be, able to suction such a settled granular material. Therefore, this prevents from the need of emptying the tank whenever the content of settled organic compound is too much. Moreover, such operation of settling the organic material prevents also an excessive concentration of such substance from being introduced into the main plant for producing biogas or biomethan. In fact, in this case more serious problems can arise in the plant with a great efficiency loss.

Unfortunately, the afore stated solution is not free from drawbacks. In fact, quite frequently the suctioning means block as accumulation of inorganic materials settled on the tank bottom is not correctly drawn. This forces to anyway empty the tank and re-establish the correct operation of the suctioning means with the consequence of cost increases and delay of biogas or biomethan production times.

### Summary of the invention

Object of the present invention is then to solve the afore mentioned problems and to provide a separating tank for the plant to separate the avicultural dejections from the inorganic portion, which guarantees a better production continuity.

A further object of the present invention is to implement a tank being also easy from a structural point of view and not needing of important modifications with respect to the base structure nowadays used for carrying out similar functions.

Finally, object of the present invention is to implement a method allowing to remove simply and without hitches the organic material settled in the tank.

These and other objects are solved by the present invention by means of a settling tank to settle the inorganic portion of the avicultural dejections, comprising at least one first chamber for settling the inorganic portion contained in said avicultural dejections, at least one bottom surface on which said inorganic portion deposits and suctioning means to suction said settled inorganic portion, characterized in that said suctioning means comprise at least one suction duct vertically movable depending on the height said inorganic portion reaches with respect to said bottom surface. According to the invention, it is possible to move the suction duct to a height able to be changed with respect to the bottom surface of the tank depending on the height reached by the settled inorganic material still with respect to the bottom surface of the tank. This allows adjusting precisely the draft height of the suction duct in order to always assure the maximum suction efficiency and without running into a snag during its operation.

In particular, according to the invention said at least one suction duct is of telescopic type. In particular, such a telescopic duct has vertical axis and upper free end, that one used for suctioning the inorganic portion lying on a substantially horizontal plane.

Moreover, said suctioning means comprise at least one mechanical actuator selected from a pneumatic, mechanical or hydraulic one, for vertically sliding said telescopic duct.

According to a preferred embodiment of the invention, said at least one actuator is integrally combined with the upper free end of said telescopic duct in order to move it vertically.

Furthermore, said tank comprises at least one level sensor for measuring the height of said settled inorganic portion with respect to said bottom surface of said at least one first settling chamber. According to an embodiment, the tank also comprises at least one control unit acquiring the value measured by said at least one level sensor and driving the movement of said at least one movable duct so that the upper free end thereof is at a distance from the bottom of said tank lower than the height reached by the settled inorganic material. Preferably, the difference between the height reached by the settled inorganic material and that one of the upper free end of the movable duct is within a range from 50 and 1000 mm.

In accordance with a preferred embodiment, the tank comprises at least one second settling chamber and at least one separating diaphragm between said at least one first settling chamber and said at least one second settling chamber; in particular, said at least one diaphragm is designed so as to allow, on top, the continuous fluidic connection between said at least one first chamber and said at least one second chamber and, on the bottom, being provided with at least one section for the controlled fluidic connection between said at least one first chamber and said at least one second chamber.

Preferably, the tank comprises at least one floodgate arranged at said passage section and movable between a first position, in which said fluidic connection between said at least one first chamber and said at least one second chamber is prevented, and a second position in which said fluidic connection between said at least one first chamber and said at least one second chamber is allowed.

In accordance with the inventive embodiments herein described, the tank comprises loading means to load said avicultural dejections into said tank, said loading means comprising at least one Venturi tube. Preferably and in accordance with the second inventive embodiment, said at least one Venturi tube is oriented towards said at least one diaphragm.

Furthermore, the afore mentioned objects are also solved by the present invention by means of a method for removing the inorganic portion settled in at least one tank according to one or more of claims 1 to 10, comprising the step of a) introducing said avicultural dejections into said at least one tank, the step of b) let said avicultural dejections settle in said at least one tank and the step of c) suctioning the inorganic portion settled on the bottom of said at least one tank, said method being characterized in that said step c) comprises the step of c1) measuring the height reached by said inorganic portion with respect to the bottom surface of said at least one tank and the step of c2) vertically changing the distance of said suction duct from said bottom surface depending on the height reached by said settled inorganic material and measured during said step c1). It has to be mentioned that the upper free end of the suction duct is at a distance from the bottom of said tank anyway lower than the height reached by the settled inorganic material.

In particular, at least when said at least one tank comprises at least one second settling chamber and at least one separating diaphragm between said at least one first settling chamber and said at least one second settling chamber, wherein said at least one diaphragm is designed so as to allow, on top, the continuous fluidic connection between said at least one first chamber and said at least one second chamber and, on the bottom, being provided with at least one section for the controlled fluidic connection between said at least one first chamber and said at least one second chamber, then said step c) of the method comprises the step of c0) moving said at least one diaphragm to said second position.

### Brief description of the drawings

Further aspects and advantages of the present invention will be more evident from the following description, made for illustration purposes only and without limitation, referring to the accompanying schematic drawings, in which:
- Figure 1 schematically shows a settling tank according to the present invention in a first embodiment;
- Figure 2 schematically shows the settling tank of figure 1 wherein the suction duct changed the distance from the tank bottom depending on the height the settled material reached with respect to the tank bottom;
- Figure 3 schematically shows a settling tank according to another embodiment;
- Figure 4 schematically shows the settling tank of figure 3 wherein the floodgate is moved to its second position;
- Figure 5 schematically shows the settling tank of figure 3 and 4 wherein the suction duct changed position depending on the height the settled material reached still with respect to the tank bottom.

### Embodiments of the invention

Referring particularly to such figures, the tank for separating the avicultural dejections according to the invention has been denoted with numeral 1. In particular, such a tank 1 is positioned upstream of a plant for the anaerobic digestion of the avicultural dejections and for the production of biogas or biomethan.

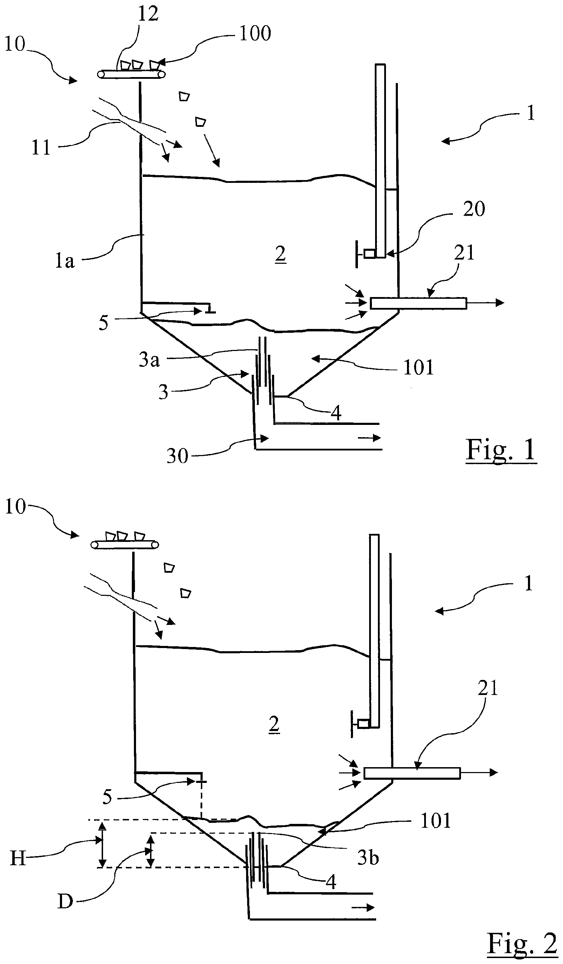

Figures 1 and 2 show a tank 1 according to a first embodiment of the invention. The tank 1 for separating the inorganic portion of the avicultural dejections 100 comprises a first chamber 2 for settling such an inorganic portion, which has a granular solid form, a bottom surface 4 on which said inorganic portion deposits and suctioning means 3 to suction such a settled granular solid inorganic material 101. According to the invention, these suctioning means 3 comprise at least one suction duct 3a vertically movable depending on the height H reached by the settled granular solid material 101 with respect to the bottom surface 4 of the tank 1. In particular, such a duct 3a has vertical axis, therefore the suction of the inorganic material occurs from top to bottom. In figure 1 the transfer line 30 is also shown, for transferring the settled inorganic portion 101 suctioned through the suction duct 3a.

According to the invention said duct 3a is of telescopic type, i.e. it does not comprise a series of pipes concentric one another and with diameter decreasing from the outermost one.

Moreover, such suctioning means 3 comprise also a mechanical actuator of the hydraulic type (herein not shown) such that the telescopic duct 3a can slide. Preferably such a hydraulic actuator, or better the movable element of such hydraulic actuator of the type for example comprising a movable piston translating with respect to the actuator cylinder, is integrally combined with the upper free end 3b of the telescopic duct 3a so that to move it vertically to a different height from the bottom 4 of the tank 1. It should be noticed that the free end lies substantially on a horizontal plane.

Anyway, it has to be noticed that the suctioning means comprising a pneumatic or mechanical actuator, rather than a hydraulic one, will fall within the protection scope of the present invention.

Still according to the embodiment herein described, the tank 1 comprises also a level sensor 5 arranged on a side wall 1a of the tank 1 to measure the height H of the settled inorganic material with respect to the bottom surface 4.

Furthermore, the tank 1 comprises a control unit (herein not shown) acquiring the value measured by the level sensor 5 and driving the vertical movement of the movable duct 3a so that the upper free end 3b thereof is at a distance D from the bottom surface 4 of the tank lower than the height H reached by the settled solid material in a range from 50 to 1000 mm, preferably from 100 to 700 mm (see figure 2). Therefore, in practice, the sensor allows measuring the height H the settled solid material 101 reached and comparing it with the distance at which the upper free end 3b of the telescopic duct 3a is. Once such values are known, it is possible to drive the movement of the free end 3b of the duct 3a so that the latter will always be in the best draft position for maintaining the appropriate suction level and no clogging could occur in the duct 3a. Figure 2 shows the settling tank 1 of figure 1, wherein the free end 3b of the suction duct 3a changed the distance D from the tank bottom 4 with respect to its own position of figure 1, as a consequence of the lowering of the height H reached by the settled material 101, still with respect to the bottom surface 4 of the tank.

Moreover, the tank 1 comprises a mixer 20 to mix continuously the avicultural dejections in the chamber 2 and to aid the separation of the organic material from the inorganic one, and an outflow pipe 21 for suctioning the organic material now almost free of inorganic material or anyway having a concentration thereof compatible with that one allowing the proper plant operation for producing biogas, or biomethan, from avicultural dejections.

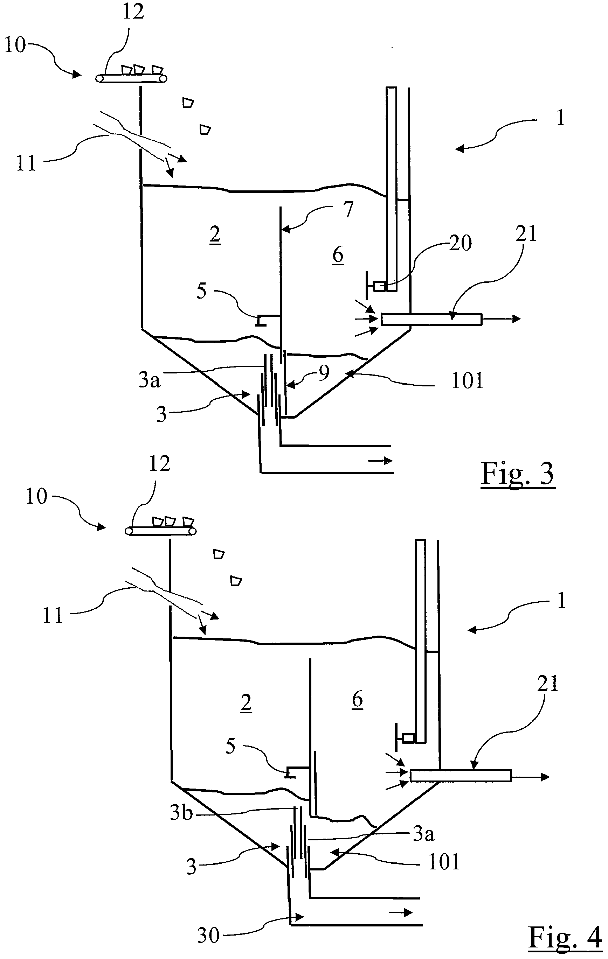

Figures 3 to 5 show the tank 1 in a second embodiment. Such a tank 1 comprises, with respect to that shown in the first embodiment of figures 1 and 2, in addition to a first settling chamber 2, also a second settling chamber 6 and a separating diaphragm 7 to separate the first settling chamber 2 and the second settling chamber 6. Such a separating diaphragm 7 is designed to allow, on top, the continuous fluidic connection between the first settling chamber 2 and the second settling chamber 6 and, on the bottom, is provided with at least one section 8 for the controlled fluidic connection between the first chamber 2 and the second chamber 6. This allows removing also the organic material in the second settling chamber 6 where, in any case, the concentration of the present inorganic material 101 will be lower than that in the first settling chamber 2.

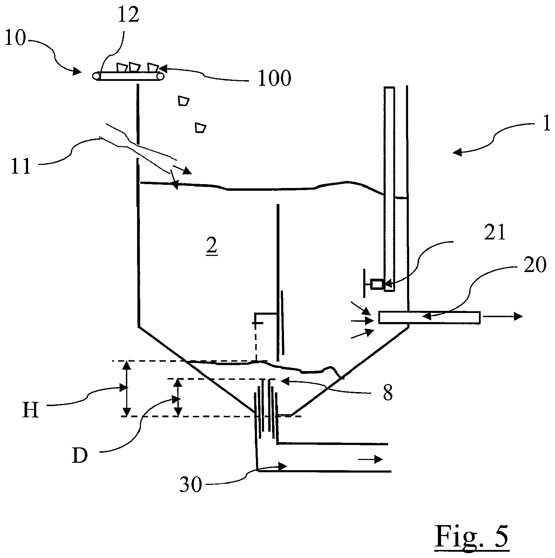

According to the particular embodiment herein depicted in figures 3 to 5, the tank 1 comprises a floodgate 9 arranged at the passage section 8 and vertically movable between a first position (see figure 3), wherein the fluidic connection between the first chamber 2 and the second chamber 6 is prevented, and a second position (see figures 4 and 5) wherein the fluidic connection between the first chamber 2 and the second chamber 6 is allowed. In figure 5, the free end 3b of the suction duct 3a lowered and changed its own distance H from the tank bottom 4, depending on the changed height D reached by the settled material, still with respect to the tank bottom 4.

In both the embodiments described so far, the tank 1 comprises loading means 10 to load the avicultural dejections into said tank 1. Such loading means 10 comprise a Venturi tube 11 and a conveyor belt 12. Advantageously, the Venturi tube 11 allows introducing the organic material into the tank 1 at high rate so that to increase the crushing degree of the material introduced in the tank 1 and to aid the subsequent step of separating and settling the inorganic material with respect to the lighter one of organic kind.

In particular it has to be clarified that, in the case of the second inventive embodiment (figures 3 to 5), the Venturi tube 11 is oriented just towards the diaphragm 7 so that the crushing effect desired with the use of the Venturi tube is increased.

According to the herein described embodiments, a method for removing the settled inorganic portion 101 in a tank 1 is provided. In the embodiments of figures 1 and 2, wherein there is only one chamber 2, such a method comprises the step of a) introducing said avicultural dejections 100 into said first settling chamber 2, the step of b) let said avicultural dejections settle in said first settling chamber 2 and the step of c) suctioning the settled inorganic material 101 on the bottom of the tank 1; advantageously, the step c) comprises the step of c1) measuring the height H reached by the settled inorganic material with respect to the bottom surface 4 of the tank 1 and the step of c2) vertically changing the distance D of the free end 3b of the suction duct 3a from the bottom surface 4 depending on the height H reached by the settled inorganic material measured during the step c1). Obviously, the upper free end 3b of the movable suction duct 3a is always at a distance D from the bottom surface 4 of said tank lower than the height H reached by said settled inorganic portion, preferably in a range from 50 to 1000 mm.

In case of the second embodiment described in figures 3 to 5, wherein the tank 1 comprises also a second settling chamber 6 and a separating diaphragm 7 arranged between the first settling chamber 2 and the second settling chamber 6 and where such a diaphragm is designed so that to allow, on top, the continuous fluidic connection between the first chamber 2 and the second chamber 6 and, on the bottom, is provided with a section 8 for the controlled fluidic connection between the first chamber 2 and the second chamber 6, the step c) of the method comprises also the step of c0) moving the floodgate 9 to its second position.(see figures 4 and 5) so as to aid even the suction of the inorganic material settled in the second settling chamber 6.

Afterwards, when all or part of the settled inorganic material 101 has been suctioned, the floodgate 9 is returned to its own first position (see figures 4 and 5) so as to prevent the fluidic connection between the first chamber 2 and the second tank 6.

## Claims

1. Settling tank (1) to settle the inorganic portion of the avicultural dejections, comprising at least one first chamber (2) for settling said inorganic portion contained in said avicultural dejections, at least one bottom surface (4) on which said inorganic portion deposits and suctioning means (3) to suction said settled inorganic portion, **characterized in that** said suctioning means (3) comprise at least one suction duct (3a) vertically movable depending on the height said settled inorganic portion reaches with respect to said bottom surface (4).

2. Tank according to claim 1, **characterized in that** said duct (3a) is of telescopic type.

3. Tank according to claim 2, **characterized in that** said suctioning means comprise at least one mechanical actuator selected from a pneumatic, mechanical or hydraulic one, for vertically moving said telescopic duct (3a).

4. Tank according to claim 3, **characterized in that** said at least one actuator is integrally combined with the upper free end (3b) of said telescopic duct (3a).

5. Tank according to one or more of claims 1 to 4, **characterized in that** said tank (1) comprises on the inside at least one level sensor (5) for measuring the height (H) of said settled inorganic portion with respect to said bottom surface (4).

6. Tank according to claim 5, **characterized by** comprising at least one control unit acquiring the value measured by said at least one level sensor (5) and driving the movement of said at least one movable duct (3a) so that the upper free end (3b) thereof is at a distance (D) from the bottom surface (4) of said tank lower than the height (H) reached by said settled inorganic portion, preferably in a range from 50 to 1000 mm.

7. Tank according to one or more of claims 1 to 6, **characterized by** comprising a second settling chamber (6) and at least one separating diaphragm (7) between said at least one first settling chamber (2) and said at least one second settling chamber (6), said diaphragm (7) being designed to allow, on top, the continuous fluidic connection between said at least one first chamber and said at least one second chamber and, on the bottom, being provided with at least one section (8) for the controlled fluidic connection between said at least one first chamber (2) and said at least one second chamber (6).

8. Tank according to claim 7, **characterized by** comprising at least one floodgate (9) arranged at said passage section (8) and movable between a first position, in which said fluidic connection between said at least one first chamber and said at least one second chamber is prevented, and a second position in which said fluidic connection between said at least one first chamber and said at least one second chamber is allowed

9. Tank according to one or more of claims 1 to 8, **characterized by** comprising loading means (10) to load said avicultural dejections into said tank (1), said loading means comprising at least one Venturi tube (11).

10. Tank according to claim 7 or 8 and 9, **characterized in that** said at least one Venturi tube is oriented towards said at least one separating diaphragm (7).

11. Plant for the anaerobic digestion of the avicultural dejections for the biogas or biomethan production, comprising at least one settling tank (1) to settle the inorganic portion of said avicultural dejections according to one or more of claims 1 to 10.

12. Method for removing the inorganic portion settled in at least one tank (1) according to one or more of the preceding claims, comprising the step of a) introducing said avicultural dejections into said at least one tank, the step of b) let said avicultural dejections settle in said at least one tank and the step of c) suctioning the inorganic portion of said dejections settled on the bottom of said at least one tank, said method being **characterized in that** said step c) comprises the step of c1) measuring the height (H) reached by said settled inorganic portion with respect to the bottom surface (4) of said at least one tank and the step of c2) vertically changing the distance (D) of said suction duct with respect to said bottom surface depending on the height reached by said settled inorganic portion measured during said step c1).

13. Method according to claim 12, **characterized in that** said step c) comprises the step of c0) moving said at least one floodgate to said second position.

## Patentansprüche

1. Absetzbecken (1) zum Absetzen des anorganischen Anteils von Hühnerhaltungs-Exkrementen, umfassend mindestens eine erste Kammer (2) zum Absetzen des in den Hühnerhaltungs-Exkrementen enthaltenen, anorganischen Anteils, mindestens eine Bodenfläche (4), auf welche sich der anorganische Anteil ablagert, und Saugmittel (3) zum Absaugen des abgesetzten, anorganischen Anteils, **dadurch gekennzeichnet, dass** die Saugmittel (3) mindestens einen Absaugkanal (3a) aufweisen, der in Abhängigkeit von der Höhe, welche der abgelagerte, anorganische Anteil in Bezug auf die Bodenfläche (4) erreicht, vertikal bewegbar ist.

2. Becken gemäß Anspruch 1, **dadurch gekennzeichnet, dass** der Kanal (3a) ein Kanal vom Teleskoptyp ist.

3. Becken gemäß Anspruch 2, **dadurch gekennzeichnet, dass** die Saugmittel zum vertikalen Bewegen des Teleskopkanals (3a) mindestens einen mechanischen Aktuator umfassen, ausgewählt aus einem pneumatischen, mechanischen oder hydraulischen Aktuator.

4. Becken gemäß Anspruch 3, **dadurch gekennzeichnet, dass** der mindestens eine Aktuator integral mit dem oberen, freien Ende (3b) des Teleskopkanals (3a) kombiniert ist.

5. Becken gemäß einem oder mehreren der Ansprüche 1 bis 4, **dadurch gekennzeichnet, dass** das Becken (1) im Inneren mindestens einen Füllstandssensor (5) umfasst, um die Höhe (H) des abgesetzten, anorganischen Anteils in Bezug auf die besagte Bodenfläche (4) zu messen.

6. Becken gemäß Anspruch 5, **dadurch gekennzeichnet, dass** es mindestens eine Kontrolleinheit umfasst, welche den von dem mindestens einen Füllstandssensor (5) gemessenen Wert erfasst und die Bewegung des mindestens einen, bewegbaren Kanals (3a) so antreibt, dass sich das obere freie Ende (3b) von der Bodenfläche (4) des Beckens in einem Abstand (D) befindet, der niedriger ist als die von dem abgesetzten, anorganischen Anteil erreichte Höhe (H), vorzugsweise in einem Bereich von 50 bis 1000 mm.

7. Becken gemäß einem oder mehreren der Ansprüche 1 bis 6, **dadurch gekennzeichnet, dass** es eine zweite Absetzkammer (6) und mindestens eine Trennmembran (7) zwischen der mindestens einen ersten Absetzkammer (2) und der mindestens einen zweiten Absetzkammer (6) umfasst, wobei die Membran (7) so ausgebildet ist, dass sie an der Spitze die dauernde, fluidische Verbindung zwischen der mindestens einen ersten Kammer und der mindestens einen zweiten Kammer ermöglicht, und dass sie am Boden mit mindestens einen Abschnitt (8) zur kontrollierten, fluidischen Verbindung zwischen der mindestens einen ersten Kammer (2) und der mindestens einen zweiten Kammer (6) ausgestattet ist.

8. Becken gemäß Anspruch 7, **dadurch gekennzeichnet, dass** es mindestens eine Schleusenklappe (9) umfasst, die am Durchlassabschnitt (8) angeordnet und bewegbar ist zwischen einer ersten Position, in welcher die fluidische Verbindung zwischen der mindestens einen ersten Kammer und der mindestens einen zweiten Kammer verhindert wird, und einer zweiten Position, in welcher die fluidische Verbindung zwischen der mindestens einen ersten Kammer und der mindestens einen zweiten Kammer zulässig ist.

9. Becken gemäß einem oder mehreren der Ansprüche 1 bis 8, **dadurch gekennzeichnet, dass** es Beladungsmittel (10) zum Beladen des Beckens (1) mit den Hühnerhaltungs-Exkrementen umfasst, wobei die Beladungsmittel mindestens ein Venturi-Rohr (11) umfassen.

10. Becken gemäß Anspruch 7 oder 8 und 9, **dadurch gekennzeichnet, dass** das mindestens eine Venturi-Rohr auf die mindestens eine Trennmembran (7) gerichtet ist.

11. Anlage zur anaeroben Vergärung von Hühnerhaltungs-Exkrementen für die Biogas- oder Biomethanproduktion, umfassend mindestens ein Absetzbecken (1) zum Absetzen des anorganischen Anteils der Hühnerhaltungs-Exkremente gemäß einem oder mehreren der Ansprüche 1 bis 10.

12. Verfahren zum Entfernen des in mindestens einem Becken (1) abgelagerten, anorganischen Anteils gemäß einem oder mehreren der vorhergehenden Ansprüche, umfassend den Schritt a) Einführen der Hühnerhaltungs-Exkremente in das mindestens eine Becken, den Schritt b) Absetzen-Lassen der Hühnerhaltungs-Exkremente in dem mindestens einen Becken, und den Schritt c) Absaugen des anorganischen Anteils der auf dem Boden des wenigstens einen Beckens abgesetzten Exkremente, wobei das Verfahren **dadurch gekennzeichnet ist, dass** der Schritt c) umfasst den Schritt c1) Messen der Höhe (H), die der abgesetzte, anorganische Anteil in Bezug auf die Bodenfläche (4) des mindestens einen Beckens erreicht hat, und den Schritt c2), vertikale Änderung des Abstands (D) des Saugkanals in Bezug auf die Bodenfläche in Abhängigkeit von der im Schritt c1) gemessenen Höhe, die der abgesetzte, anorganische Anteil erreicht hat.

13. Verfahren gemäß Anspruch 12, **dadurch gekennzeichnet, dass** der Schritt c) den Schritt c0) umfasst, nämlich das Bewegen der mindestens einen Schleusenklappe in die zweite Position.

## Revendications

1. Réservoir de décantation (1) pour décanter la partie inorganique des déjections avicoles, comprenant au moins une première chambre (2) pour la décantation de ladite partie inorganique contenue dans lesdites déjections avicoles, au moins une surface inférieure (4) sur laquelle ladite partie inorganique se dépose et un moyen d'aspiration (3) pour aspirer ladite partie inorganique décantée, **caractérisé en ce que** ledit moyen d'aspiration (3) comprend au moins une conduite d'aspiration (3a) mobile verticalement en fonction de la hauteur que ladite partie inorganique décantée atteint par rapport à ladite surface inférieure (4).

2. Réservoir selon la revendication 1, **caractérisé en ce que** ladite conduite (3a) est de type télescopique.

3. Réservoir selon la revendication 2, **caractérisé en ce que** ledit moyen d'aspiration comprend au moins un actionneur mécanique choisi parmi un pneumatique, mécanique ou hydraulique, pour déplacer verticalement ladite conduite télescopique (3a).

4. Réservoir selon la revendication 3, **caractérisé en ce que** ledit au moins un actionneur est combiné d'un seul tenant avec l'extrémité libre supérieure (3b) de ladite conduite télescopique (3a).

5. Réservoir selon une ou plusieurs des revendications 1 à 4, **caractérisé en ce que** ledit réservoir (1) comprend à l'intérieur au moins un capteur de niveau (5) pour mesurer la hauteur (H) de ladite partie inorganique décantée par rapport à ladite surface inférieure (4).

6. Réservoir selon la revendication 5, **caractérisé en ce qu'**il comprend au moins une unité de commande acquérant la valeur mesurée par ledit au moins un capteur de niveau (5) et entraînant le mouvement de ladite au moins une conduite mobile (3a) de sorte que l'extrémité libre supérieure (3b) de celle-ci est à une distance (D) de la surface inférieure (4) dudit réservoir inférieure à la hauteur (H) atteinte par ladite partie inorganique décantée, de préférence dans une plage de 50 à 1 000 mm.

7. Réservoir selon une ou plusieurs des revendications 1 à 6, **caractérisé en ce qu'**il comprend une seconde chambre de décantation (6) et au moins un diaphragme de séparation (7) entre ladite au moins une première chambre de décantation (2) et ladite au moins une seconde chambre de décantation (6), ledit diaphragme (7) étant conçu pour permettre, en haut, la connexion fluidique continue entre ladite au moins une première chambre et ladite au moins une seconde chambre et, au fond, étant doté d'au moins une section (8) pour la connexion fluidique commandée entre ladite au moins une première chambre (2) et ladite au moins une seconde chambre (6).

8. Réservoir selon la revendication 7, **caractérisé en ce qu'**il comprend au moins une vanne de décharge (9) agencée au niveau de ladite section de passage (8) et mobile entre une première position, dans laquelle ladite connexion fluidique entre ladite au moins une première chambre et ladite au moins une seconde chambre est empêchée, et une seconde position dans laquelle ladite connexion fluidique entre ladite au moins une première chambre et ladite au moins une seconde chambre est autorisée.

9. Réservoir selon une ou plusieurs des revendications 1 à 8, **caractérisé en ce qu'**il comprend un moyen de chargement (10) pour charger lesdites déjections avicoles dans ledit réservoir (1), ledit moyen de chargement comprenant au moins un tube de Venturi (11).

10. Réservoir selon la revendication 7 ou 8 et 9, **caractérisé en ce que** ledit au moins un tube de Venturi est orienté vers ledit au moins un diaphragme de séparation (7).

11. Usine pour la digestion anaérobie des déjections avicoles pour la production de biogaz ou de biométhane, comprenant au moins un réservoir de décantation (1) pour décanter la partie inorganique desdites déjections avicoles selon une ou plusieurs des revendications 1 à 10.

12. Procédé pour éliminer la partie inorganique décantée dans au moins un réservoir (1) selon une ou plusieurs des revendications précédentes, comprenant l'étape a) consistant à introduire lesdites déjections avicoles dans ledit au moins un réservoir, l'étape b) consistant à laisser décanter lesdites déjections avicoles dans ledit au moins un réservoir et l'étape c) consistant à aspirer la partie inorganique desdites déjections décantées au fond dudit au moins un réservoir, ledit procédé étant **caractérisé en ce que** ladite étape c) comprend l'étape c1) consistant à mesurer la hauteur (H) atteinte par ladite partie inorganique décantée par rapport à la surface inférieure (4) dudit au moins un réservoir et l'étape c2) consistant à changer verticalement la distance (D) de ladite conduite d'aspiration par rapport à ladite surface inférieure en fonction de la hauteur atteinte par ladite partie inorganique décantée mesurée durant ladite étape c1).

13. Procédé selon la revendication 12, **caractérisé en ce que** ladite étape c) comprend l'étape c0) consistant à déplacer ladite au moins une vanne de décharge jusqu'à ladite seconde position.
